# EUROPEAN PATENT SPECIFICATION

(11) **EP 0 521 304 B1**
(45) Date of publication and mention of the grant of the patent: **28.08.1996**
(21) Application number: 92109282.1
(22) Date of filing: 02.06.1992
(51) Int. Cl.: G01N 33/50, G01N 33/543

(54) **An impervious article with a microporous surface and process for its production**
Undurchlässiger Artikel mit mikroporöser Oberfläche und Verfahren zur Herstellung desselben
Article imperméable avec une surface microporeuse et procédé pour sa fabrication

(30) Priority: 03.07.1991 GB 9114429
(43) Date of publication of application: 07.01.1993
(73) Proprietor: PALL CORPORATION, Glen Cove, New York 11542 (US)
(72) Inventor: Page, Roger Dr., Hayling Isl., Hants. P011 0BB (GB); Quinlan, Kevin M. Dr., Idsworth House, Idsworth , P08 0AW (GB)
(74) Representative: Dost, Wolfgang, Dr.rer.nat., Dipl.-Chem.

(56) References cited:
- EP-A- 417 525
- EP-A- 0 005 536
- EP-A- 0 087 228
- EP-A- 0 090 483
- EP-A- 0 274 911
- WO-A-86/07544

## Description

This invention relates to an impervious article with a microporous surface and a process for its production.

In many laboratories and diagnostic test systems, it is necessary to apply small volumes of different analyte containing solutions or reagents to closely adjacent areas on a microporous carrier or reaction medium. The microporous medium provides an appropriate large surface area with defined chemical properties in which subsequent binding and detection chemistries can be performed. Microporous polyamide membranes comprising a layer of microporous nylon membrane with a typical thickness of 140µm and with a range of porosity from 0.1µm to greater than 5.0µm have been used widely in nucleic acid transfer techniques and diagnostic test systems. Traditionally those microporous nylon membranes are skinless, flexible and equally microporous from either side and will permit both lateral and transverse capillary flow of a wide variety of fluids.

Such skinless membranes are already known from EP-A-0 005 536. There a microporous hydrophilic skinless alcohol-insoluble membrane sheet of polyhexamethylene adipamide and a process for its production are described.

However there are a large mumber of possible applications where the membrane should be impervious and more rigid. This could be accomplished by, for example, attaching it to an impervious support medium by a variety of means including heat welding, ultrasonic welding and adhesive bonding. The resulting device would be easier to handle and can be presented in a variety of shapes and forms appropriate to the laboratory test or diagnostic procedure employed. Membranes to which rigidity is imparted in such a manner suffer from a number of deficiencies which include destruction of the microporosity of the membrane at points of attachments and imperfect adhesion leaving gaps between the membrane and rigid support. Also if small volumes of solutions are applied in close proximity, lateral capillary flow will result in cross mixing of the reagents; this limits the amount and density of spacing of different reagents.

An object of the present invention is to provide a rigid impervious article, in particular in form of a sheet with a microporous surface without the aforementioned disadvantages such as loss of microporosity caused by the adhesive method, or chemical interference of the used adhesives.

A further object of the present invention is to provide a process for preparing such an article or sheet.

The present invention resides in an article as defined in the claims.

The microporous phase or layer and the solid, impervious phase or layer of the preferred sheet are bound through an area of enmeshing or blending of their respective molecules without extraneous bonding materials such as adhesives.

In accordance with the present invention there is also provided a process as defined in the claims for preparing such an article. The article of this invention comprises an area between the microporous structure and the support in which area the molecules from the original microporous structure and the original support are enmeshed. Support and microporous layer can be referred to as being "solution-welded". The preferred polymers useful for this invention are polyamides (PA), in particular nylon 6,6, nylon 6, nylon 4,6, nylon 6,10, and polyvinylidene difluoride (PVDF). The respective preferred solvents are formic acid for polyamide and dimethylformamide (DMF) for PVDF.

Preferred embodiments are the subjects of the dependent claims.

The present invention is in its preferred embodiment a new and further development of the known porous materials described in US-A-4 340 479 and US-A-4 707 266. In accordance with the present invention there is a specific bond between preferably a microporous alcohol-insoluble, hydrophilic and skinless polyamide area which returns to hydrophobicity (no longer being wetted by water) when heated just below its softening point, and which is attached directly to a non-porous solid support to form the article or preferably sheet. Adhesives or other extraneous bonding substances are absent in a connecting area between the microporous or membrane area and the support.

The microporous structure is free of an extraneous substrate; in particular there is no woven or unwoven fabric as a substrate for the microporous structure. Rather, the microporous structure is as such directly and without adhesives by common solvent solution bonding connected to the impervious support.

Preferably the bond is created by a process which comprises the steps of preparing a casting solution of polyamide in formic acid, adding a prescribed quantity of water with controlled mixing thereby inducing nucleation of the solution, introducing a sheet of the nucleated solution into contact with a solid support and thereafter into a quench bath thereby forming a structure or film of the nucleated solution on the support surface. The molecules of the microporous structure or layer are enmeshed by forming a liquid or fluid with the support surface and the bond is established during re-solidification thereof. Frequently, the resolidified enmeshed molecules form a microporous layer between support and microporous structure with a visibly different pore structure.

The exposed surface of the article is preferably naturally hydrophilic (i.e. without such additives as surfactants) and skinless. Thus a drop of water in contact with the surface loses its form within one second or less. The surface is also unbroken (which can be demonstrated by means of a Scanning Electron Microscope - SEM) which is important when the article is used as an analytical tool.

The quenching stage of the above mentioned process may be omitted, allowing the casting solvent to be evaporated. Articles prepared by this route tend to have less uniform pore structures but are nevertheless microporous on their surface.

The connecting area between the microporous structure or area and the solid impervious support is formed by "solution-welding". This means the in-situ formation of a chemical solvent bond enmeshing the molecules of the two layers, namely the microporous layer and the impervious layer or support layer.

The term "impervious" means that the impervious structure (or preferably sheet) has, at least in its area of use where it is connected to the microporous structure, a surface which has no through pores or voids, or a structure which will not allow water or alcohol to pass through.

The preferred thickness of the microporous structure or layer is from 10µm to 200µm. The preferred thickness of the solid impervious support is from 100µm to 3mm. The preferred thickness of the inter-connecting area lies within the range 10µm to 40µm.

Preferably the microporous structure or layer and the solid impervious support are made of like material, i.e. material soluble in a common solvent, particularly the solvent useful for forming a casting solution of the polymer for making the microporous structure. More particularly, the two portions of the articles may be made of the same material. This material may be selected from nylon 6, nylon 4,6, nylon 6,6 and nylon 6,10. Dissimilar polymers may be used provided they have a common solvent (like materials).

In a preferred embodiment of the invention biochemical reactants, preferably polyclonal or monoclonal antibodies or antigens are attached to the internal surfaces of the microporous structure or area of the sandwich structure of microporous structure, interconnecting layer and solid, impervious support.

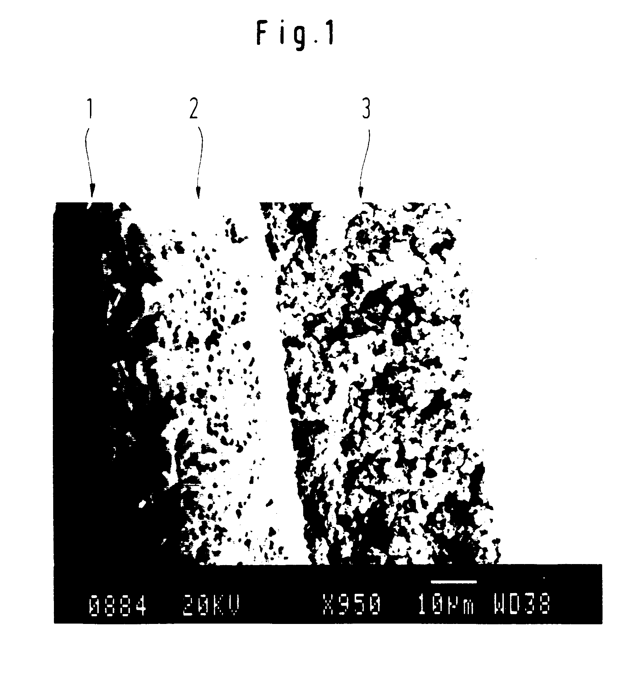

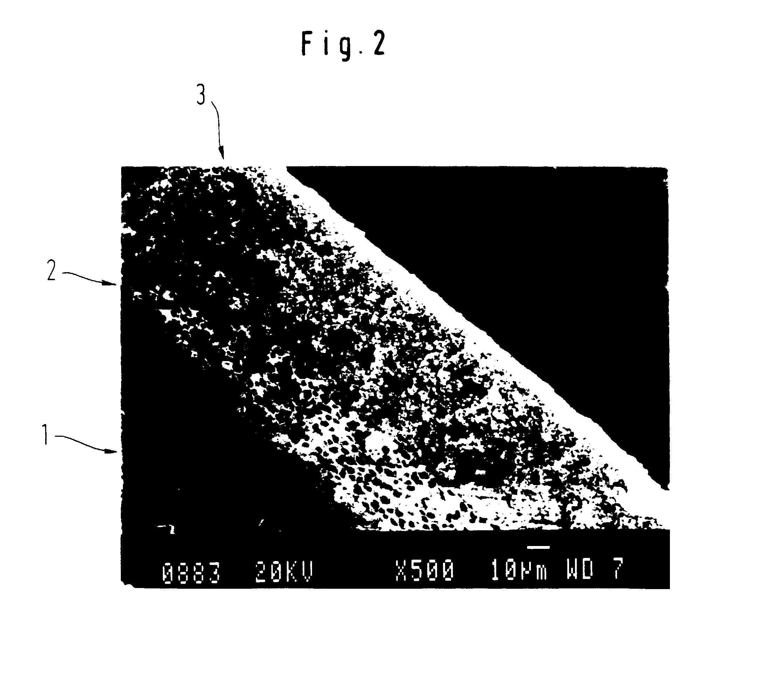

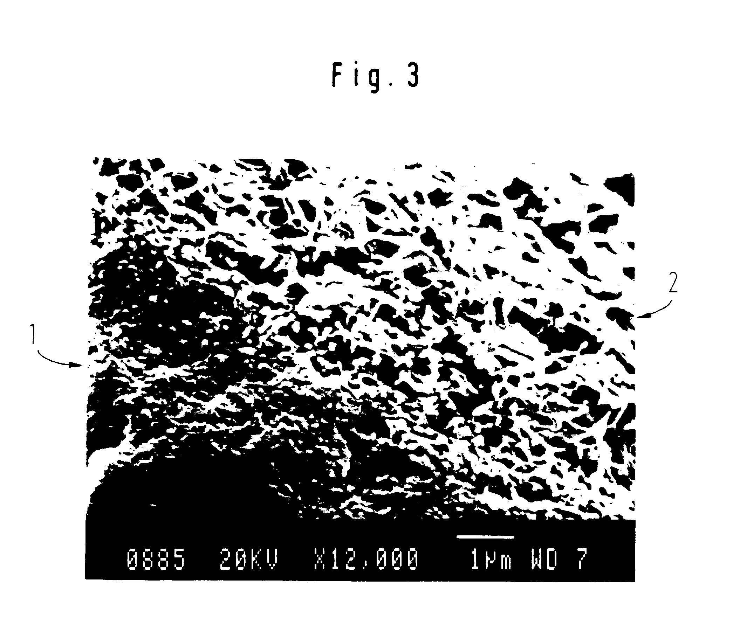

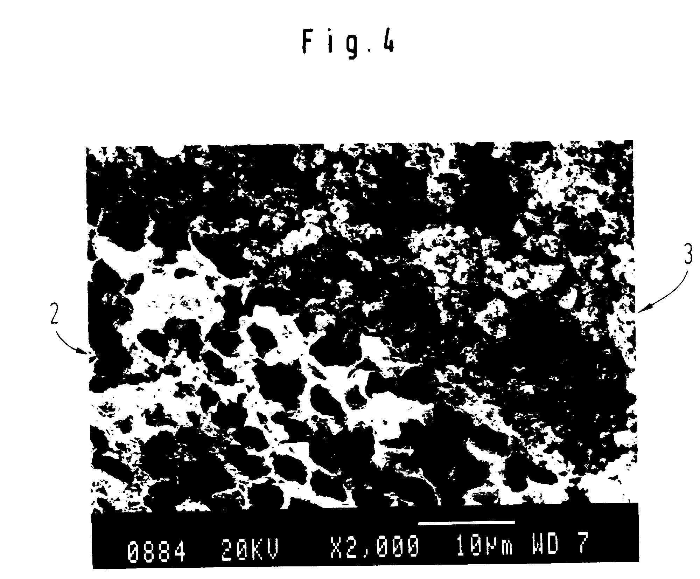

The invention and preferred features and embodiments thereof are described in connection with the drawing in which Figs. 1 and 2 each show a copy from an SEM photograph of a cross section of a composite sheet. Figs. 3 and 4 each show a copy from an SEM photograph which is an enlargement of a portion of the photograph shown in Fig. 2.

Figs. 1 and 2 show that the sheet has effectively three layers. On the left there is solid nylon 1 (support), on the right directly cast un-skinned microporous hydrophilic nylon 3 (microporous area) and the central zone is of induced Porosity 2 (porous area) where the solvent of the casting solution has dissolved and resolidified the support. The porous intermediate area 2 forms a distinct region between the solid support and the microporous area. The border visible in an SEM of a cross section exists between the porous area 2 and the microporous area 3 (Figs. 1, 2 and 4). A visible border can also be seen between the porous intermediate area 2 (induced porosity) and the solid support 1 in Figs. 1, 2 and 3.

The surface of the article (preferably of the sheet) of the present invention is skinless and hydrophilic on at least one side. It can be confined to selected areas of the substrate by masking during construction or by subsequent mechanical or thermal modification. It can be chemically modified to produce a charge on the pore surfaces of the microporous area by pre- or post-casting additives.

By controlled variation of the polymer, all or any of the substrate, the polymer concentration, the solvent system of the casting solution, the composition of the quench bath, the time of quenching, the quench bath temperature, additives to the quench bath, and/or the casting solution, the properties of the resulting surface can be readily varied.

After quenching, the articles can be further processed or treated in accordance with their intended use. For example, the surfaces may be washed to remove the process liquids and dried. The article may be subsequently cut or formed into devices for particular applications.

Using the procedure of casting the microporous nylon onto a rigid impervious nylon support whereby a chemical solvent bond enmeshes the molecules between the two layers overcomes the problem of adhesion and loss of microporosity described above. This solution bond is also resistant to extreme conditions during subsequent processing in laboratory or diagnostic tests. Examples of these would be repetitive cycles of up to one hour of oven baking or autoclaving at 121 °C; boiling in dilute aqueous reagent solutions (e.g. 0.5% sodium dodecyl sulfate) and heating in concentrated reagent solutions at temperatures up to 65 °C (e.g. 75% formamide or 0.5M sodium hydroxide).

The properties of the preferred hydrophilic microporous nylon enable the creation of permanent hydrophobic areas by thermal treatment, i.e. heating at just below its melting point of the nylon so that the membrane structure in those heated areas returns to hydrophobicity while remaining microporous, or preferably melting using hot shoe or ultrasonic techniques or other localized beating methods. Normally this would distort thin flexible non-rigidly supported microporous nylon. Very precise thermal images can be transferred to the microporous surface of the above article and its geometry maintained during the subsequent aggressive process conditions previously described. Typical thermal images are grid like patterns, formed with 1 mm or 1.5 mm lines enclosing 2 mm or 3 mm square areas of hydrophilic microporous nylon. The melted grid lines are sufficiently deep to form a trough deeper than the thickness of the microporous layer by penetrating into the rigid nylon support layer. This forms a hydrophobic impervious barrier between each adjacent microporous reaction zone and makes possible the production of such zones of very high density without the risk of cross mixing of applied analytes or reagents. A much larger number of tests can therefore be obtained per unit area of microporous nylon employed.

The invention will be further described by the following examples, which are provided to illustrate but not to limit the scope of the present invention.

### EXAMPLE 1

Nylon 66 resin pellets of molecular weight averaging about 40,000 were dissolved in 98.8% formic acid to yield a 30 °C solution containing 15% by weight nylon. Without delay the solution was delivered to an in-line mixer at a flow rate of 1,000 g per minute while simultaneously feeding a flow of water of 30 g/minute at 30 °C. The casting resin solution thus formed was fed through a 10 µm filter to remove visible solids. Without delay solid tabs of nylon 66 (20 x 100 x 3 mm) were partially immersed in the casting resin solution for 5 seconds. Each tap was allowed to drain for 10 seconds and excess resin was wiped off by means of a metal blade. The tabs were immediately immersed in 52% formic acid solution for 5 minutes, rinsed in deionized water for 10 minutes and dried in air for 24 hours at 30 °C.

### EXAMPLE 2

Casting resin solution was prepared as in Example 1 but Kymene resin was included to contribute 1.75 wt.% of the total solids (nylon and Kymene = 100%). Tabs produced from this resin as described in Example 1 exhibited a positive charge on the microporous surface of the composite.

### EXAMPLE 3

Sheets prepared according to the present invention were used in a sandwich Enzyme Linked Immunosorbent Assay (ELISA) in which an antigen is sandwiched between two antibodies. A capture antibody (Goat-anti-Rabbit immunoglobulin) was loaded onto several discrete and specific areas on the microporous surface of a sheet. Unused chemical binding sites on the sheet were blocked by application of casein. An antigen (Rabbit-anti-Mouse immunoglobulin) was allowed to bind to the captured antibodies and subsequently sandwiched by a second antibody linked to an enzyme (Goat-anti-Rabbit immunoglobulin - horseradish peroxidase conjugate). Excess and unbound reagents were removed by rinsing. The resultant sheet-bound complex was readily detected by application of diamino benzidine which on combination with the horseradish peroxidase formed a brown colored compound. The colored dots were discrete, round, uniform in color and easy to see.

### EXAMPLE 4

Casting resin solution was prepared as in Example 1. Small (1mm²) dots were deposited by means of a hypodermic syringe into plates of various materials and dried in air. The dried deposits were microporous and their adhesion to the various substrates ranged from
- weakly bonded: Polypropylene and polymethylmethacrylate, to
- medium bonded: Polystyrene, polycarbonate, polyester, polyvinylacetate, and
- strongly bonded: Nylon 66.

## Claims

1. An impervious article comprising
- an impervious polymeric support
- a microporous, polymeric structure
-- attached to this support by enmeshed polymer molecules from said support and from said microporous structure,
-- said microporous structure being free of any extraneous substrate.

2. An impervious article according to claim 1 characterized by a microporous alcohol-insoluble polyamide enmeshed with a like polymeric solid support.

3. An impervious article according to claim 1 or 2 characterized by the microporous, polymeric structure being hydrophilic and skinless at its outer surface.

4. An impervious article according to any of claims 1 to 3 characterized by polymer chains of the microporous, polymeric structure being enmeshed with those of the impervious polymeric support by partial dissolution of the support by which the casting solvent of the microporous, polymeric structure creates a porous layer between the microporous polymeric structure and the impervious, polymeric structure.

5. An impervious article according to claim 4 characterized by:
- the said porous layer forming a distinct region between the impervious polymeric support and the microporous, polymeric structure; and
- the said porous layer is porous but with pores distinctive to those of the microporous, polymeric structure; and
- a border exists between the porous layer and the microporous, polymeric structure.

6. An impervious article according to any of claims 1 to 5, wherein the microporous, polymeric structure and the impervious polymeric support are from a chemically similar material.

7. An impervious article according to claim 6, wherein said material is selected from nylon 6,6, nylon 4,6, nylon 6, nylon 6,10.

8. An impervious article according to any of claims 1 to 7 wherein said microporous, polymeric structure has pores having a pore size between 0.04µm and 5µm.

9. An impervious article according to any of claims 1 to 8 having a microporous, polymeric structure of a thickness between 10µm to 200µm preferably 10µm to 40µm, and/or having an impervious polymeric support of a thickness between 100µm to 3mm.

10. An impervious article according to any of claims 1 to 9, characterized by biochemical reactants, preferably polyclonal or monoclonal antibodies or antigens attached to the walls of the pores of the said microporous, polymeric structure.

11. Article according to one of the preceeding claims wherein said impervious polymeric support is in the form of an impervious sheet carrying on at least one of its surfaces at least one layer of said microporous, polymeric structure.

12. Article in accordance with one of the preceeding claims characterized in that,
a) said impervious support carries at least one but, preferably a large number of, microporous polymeric structures on its impervious surface;
b) said microporous, polymeric structures are separated from each other by surrounding regions preventing the flow of at least one fluid, preferably water, from one microporous, polymeric structure to another, said regions being preferably hydrophobic microporous regions, molten regions of microporous, polymeric structure, which therefore are no longer microporous, or regions with no microporous polymeric structure at all.

13. Article of claim 12 wherein said microporous polymeric structures are arranged in a geometrical pattern, preferably on a rectangular grid or on a grid of concentric circles, and wherein the individual structure has a surface extension covering about 1 to about 200 mm² of the surface of the impervious support.

14. A process for preparing an impervious article according to any of the preceeding claims:
a) comprising contacting a nucleated solvent based polymer casting mixture and an impervious polymeric support which is at least partially soluble in said solvent,
b) allowing said casting mixture to form a microporous, polymeric structure on said support and enabling the polymer chains of the forming microporous, polymeric structure and those of the support to enmesh,
c) removing the solvent to form the impervious article.

15. A process according to claim 14, comprising the steps of preparing a casting solution of polyamide in formic acid, adding a prescribed quantity of water with controlled mixing thereby inducing nucleation of the solution, introducing a thin layer or film of said solution into contact with said impervious, polymeric support and converting said thin layer or film of said solution into a microporous membrane structure by contacting it with a quench bath of non-solvent, washing and drying the impervious article so formed.

16. A process according to claim 14 or 15 in which the impervious, polymeric support is a non-porous synthetic polyamide film having a surface that is wetted by the casting solution.

## Patentansprüche

1. Ein undurchlässiger Gegenstand umfassend
- einen undurchlässigen polymeren Träger
- eine mikroporöse, polymere Struktur
-- die an diesem Träger durch vermischte polymere Moleküle des Trägers und der mikroporösen Struktur angebracht ist,
-- wobei die mikroporöse Struktur frei von jeglichen Fremdsubstraten ist.

2. Undurchlässiger Gegenstand nach Anspruch 1, dadurch gekennzeichnet, daß ein mikroporöses alkohol-unlösliches Polyamid mit einem gleichen polymeren festen Träger vermischt ist.

3. Undurchlässiger Gegenstand nach einem der Ansprüche 1 oder 2, dadurch gekennzeichnet, daß die mikroporöse, polymere Struktur an ihrer Außenfläche hydrophil und hautlos ist.

4. Undurchlässiger Gegenstand nach einem der Ansprüche 1 bis 3, dadurch gekennzeichnet, daß polymere Ketten der mikroporösen, polymeren Struktur mit denen des undurchlässigen polymeren Trägers durch teilweise Dissolution des Trägers vermischt wird, wodurch die Gießlösung der mikroporösen, polymeren Struktur eine poröse Schicht zwischen der mikroporösen, polymeren Struktur und der undurchlässigen polymeren Struktur bildet.

5. Undurchlässiger Gegenstand nach Anspruch 4, dadurch gekennzeichnet, daß :
- die poröse Schicht, einen bestimmten Bereich zwischen dem undurchlässigen, polymeren Träger und der mikroporösen, polymeren Struktur bildet; und
- die poröse Schicht porös ist, aber mit Poren, die unterscheidbar sind zu denen der mikroporösen, polymeren Struktur; und
- eine Grenze zwischen der porösen Schicht und der mikroporösen, polymeren Struktur existiert.

6. Undurchlässiger Gegenstand nach einem der Ansprüche 1 bis 5, dadurch gekennzeichnet, daß die mikroporöse, polymere Struktur und der undurchlässige polymere Träger aus einem chemisch ähnlichen Material sind.

7. Undurchlässiger Gegenstand nach Anspruch 6, wobei das Material aus Nylon 6,6, Nylon 4,6, Nylon 6 und Nylon 6,10 ausgewählt ist.

8. Undurchlässiger Gegenstand nach einem der Ansprüche 1 bis 7, dadurch gekennzeichnet, daß die mikroporöse, polymere Struktur Poren aufweist, die eine Porengröße zwischen 0,04µm und 5µm aufweisen.

9. Undurchlässiger Gegenstand nach einem der Ansprüche 1 bis 8, der eine mikroporöse, polymere Struktur einer Dicke zwischen 10µm bis 200 µm, vorzugsweise 10µm bis 40µm, aufweist und /oder der einen undurchlässigen polymeren Träger einer Dicke zwischen 100µm bis 3mm aufweist.

10. Undurchlässiger Gegenstand nach einem der Ansprüche 1 bis 9, dadurch gekennzeichnet, daß biochemische Reaktanden, vorzugsweise polyklonale oder monoklonale Antikörper oder Antigene an den Wänden der Poren der mikroporösen, polymeren Struktur angebracht sind.

11. Gegenstand nach einem der vorangehenden Ansprüche, dadurch gekennzeichnet, daß der undurchlässige polymere Träger in Gestalt eines undurchlässigen Blatts ausgebildet ist, das an mindestens einer seiner Oberflächen mindestens eine Schicht der mikroporösen, polymeren Struktur trägt.

12. Gegenstand nach einem der vorhergehenden Ansprüche, dadurch gekennzeichnet, daß
a) der undurchlässige Träger mindestens eine, aber vorzugsweise eine große Anzahl von mikroporösen, polymeren Strukturen an seiner undurchlässigen Oberfläche trägt;
b) die mikroporösen, polymeren Strukturen voneinander durch umgebende Bereiche getrennt sind, wobei der Fluß von mindestens einer Flüssigkeit, bevorzugt Wasser, von einer mikroporösen, polymeren Struktur zu einer anderen verhindert wird, wobei die Bereiche vorzugsweise hydrophobe mikroporöse Bereiche, geschmolzene Bereiche von mikroporösen, polymeren Strukturen, die deshalb nicht länger mikroporös sind, oder Bereiche ohne jegliche mikroporöse, polymere Struktur sind.

13. Gegenstand nach Anspruch 12, dadurch gekennzeichnet, daß mikroporöse, polymere Strukturen in einem geometrischen Muster, bevorzugt in einem rechteckigen Gitter oder in einem Gitter von konzentrischen Kreisen angeordnet sind und wobei die individuelle Struktur eine aufgespannte Oberfläche aufweist, die ungefähr 1 bis ungefähr 200 mm² der Oberfläche des undurchlässigen Trägers abdeckt.

14. Verfahren zur Herstellung eines undurchlässigen Gegenstands nach einem der vorangehenden Ansprüche:
a) umfassend das in-Kontakt-bringen einer polymeren Gießmischung, die auf einer keimbildenden Lösung basiert, und einem undurchlässigen polymeren Träger, der mindestens teilweise in der Lösung lösbar ist,
b) Gestatten der Gießmischung einer mikroporöse, polymere Struktur an dem Träger zu bilden und Ermöglichen der polymeren Ketten der sich bildenden mikroporösen, polymeren Struktur und denen des Trägers sich zu vermischen,
c) Entfernen der Lösung, um den undurchlässigen Gegenstand zu bilden.

15. Verfahren nach Anspruch 14, umfassend die Schritte des Herstellens einer Gießlösung aus Polyamiden in Ameisensäure, des Zusetzens einer vorgeschriebenen Menge von Wasser mit kontrolliertem Mischen, wodurch die Keimbildung der Lösung induziert wird, des Einführens einer dünnen Schicht oder eines Films der Lösung in Kontakt mit dem undurchlässigen polymeren Träger und des Umwandelns dieser dünnen Schicht oder des Films der Lösung in eine mikroporöse Membranstruktur durch in Berührung bringen mit einem Quenchbad von Nicht-Lösungsmittel, waschen und trocknen des so gebildeten undurchlässigen Gegenstands.

16. Verfahren nach einem der Ansprüche 14 oder 15, bei dem der undurchlässige polymere Träger ein nicht-poröser, synthetischer Polyamidfilm ist, der eine Oberfläche aufweist, die durch die Gießlösung benäßt ist.

## Revendications

1. Article imperméable comportant :
- un support imperméable en polymère
- une structure microporeuse en polymère
- fixée sur ce support par entrelacement des molécules de polymère dudit support et de ladite structure microporeuse,
- ladite structure microporeuse étant exempt de substrat étranger.

2. Article imperméable selon la revendication 1, caractérisé en ce que le polyamide microporeux insoluble dans l'alcool est entrelacé avec un support solide en polymère analogue.

3. Article imperméable selon la revendication 1 ou 2, caractérisé en ce que la structure microporeuse en polymère est hydrophile et sans peau au niveau de sa surface extérieure.

4. Article imperméable selon l'une quelconque des revendications 1 à 3, caractérisé en ce que des chaînes de polymère de la structure microporeuse en polymère sont entrelacées avec celles du support imperméable en polymère par dissolution partielle du support par laquelle le solvant de coulage de la structure microporeuse en polymère crée une couche poreuse entre la structure microporeuse en polymère et la structure imperméable en polymère.

5. Article imperméable selon la revendication 4, caractérisé en ce que :
- ladite couche poreuse forme une zone distincte entre le support imperméable en polymère et la structure microporeuse en polymère ; et
- ladite couche poreuse est poreuse mais en ayant des pores distincts de ceux de la structure microporeuse en polymère ; et
- une limite existe entre la couche poreuse et la structure microporeuse en polymère.

6. Article imperméable selon l'une quelconque des revendications 1 à 5, dans lequel la structure microporeuse en polymère et le support imperméable en polymère sont constitués d'un matériau chimiquement similaire.

7. Article imperméable selon la revendication 6, dans lequel ledit matériau est sélectionné parmi le nylon 6,6, le nylon 4,6, le nylon 6, le nylon 6,10.

8. Article imperméable selon l'une quelconque des revendications 1 à 7, dans lequel ladite structure microporeuse en polymère a des pores ayant une dimension de pore comprise entre 0,04 µm et 5 µm.

9. Article imperméable selon l'une quelconque des revendications 1 à 8 comportant une structure microporeuse en polymère ayant une épaisseur comprise ente 10 µm et 200 µm, de préférence entre 10 µm et 40 µm, et/ou comportant un support imperméable en polymère ayant une épaisseur comprise entre 100 µm et 3 mm.

10. Article imperméable selon l'une quelconque des revendications 1 à 9, caractérisé en ce que des réactifs biochimiques, de préférence des anticorps ou des antigènes polyclonaux ou monoclonaux, sont fixés sur les parois des pores de ladite structure microporeuse en polymère.

11. Article selon l'une quelconque des revendications précédentes, dans lequel ledit support imperméable en polymère a la forme d'une feuille imperméable supportant sur au moins une de ses surfaces au moins une couche de ladite structure microporeuse en polymère.

12. Article selon l'une quelconque des revendications précédentes, caractérisé en ce que
a) ledit support imperméable supporte au moins une, mais de préférence un nombre important, de structure microporeuse en polymère sur sa surface imperméable ;
b) lesdites structures microporeuses en polymère sont séparées les unes des autres par des zones entourantes empêchant l'écoulement d'au moins un fluide, de préférence de l'eau, à partir d'une structure microporeuse en polymère vers une autre, lesdites zones étant de préférence des zones microporeuses hydrophobes, des zones fondues de structure microporeuse en polymère, qui par conséquent ne sont plus microporeuses, ou des zones n'ayant pas du tout de structure microporeuse en polymère.

13. Article selon la revendication 12 dans lequel lesdites structures microporeuses en polymère sont agencées dans un motif géométrique, de préférence sur une grille rectangulaire ou sur une grille de cercles concentriques, et dans lequel la structure individuelle a une étendue superficielle recouvrant environ 1 à 200 mm² de la surface du support imperméable.

14. Procédé pour préparer un article imperméable selon l'une quelconque des revendications précédentes consistant à :
a) mettre en contact un mélange de coulage de polymère à base de solvant nucléé et un support imperméable en polymère qui est au moins partiellement soluble dans ledit solvant,
b) permettre audit mélange de coulage de former une structure microporeuse en polymère sur ledit support et permettre l'entrelacement des chaînes de polymère de la structure microporeuse en polymère en formation et de celles du support,
c) enlever le solvant pour former l'article imperméable.

15. Procédé selon la revendication 14, comportant les étapes consistant à préparer une solution de coulage de polyamide dans l'acide formique, ajouter une quantité prescrite d'eau avec mélange commandé de manière à commencer la nucléation de la solution, mettre une fine couche ou un film de ladite solution en contact avec ledit support imperméable en polymère et convertir ledit film ou ladite couche fine de ladite solution en une structure de membrane microporeuse par mise en contact de celle-ci avec un bain de trempage de non-solvant, nettoyer et sécher l'article imperméable ainsi formé.

16. Procédé selon la revendication 14 ou 15 dans lequel le support imperméable en polymère est un film de polyamide synthétique non poreux ayant une surface qui est humectée par la solution de coulage.
